# EUROPEAN PATENT SPECIFICATION

(11) **EP 4 476 920 B1**
(45) Date of publication and mention of the grant of the patent: **09.12.2026**
(21) Application number: 23749410.9
(22) Date of filing: 02.02.2023
(51) Int. Cl.: H04R 25/00, G06F 3/16, H04R 29/00, A61N 1/05, A61N 1/36, A61N 1/372

(54) **BALANCED HEARING DEVICE LOUDNESS CONTROL**
AUSGEGLICHENE LAUTSTÄRKEREGELUNG FÜR HÖRGERÄT
RÉGULATION DE L'INTENSITÉ SONORE DE DISPOSITIF AUDITIF ÉQUILIBRÉE

(30) Priority: 07.02.2022 US 202263307319 P
(43) Date of publication of application: 18.12.2024
(73) Proprietor: Cochlear Limited, Macquarie University, New South Wales 2109 (AU)
(72) Inventor: VAN DIJK, Bastiaan, Macquarie University, New South Wales 2109 (AU); ARTASO, Miguel, Macquarie University, New South Wales 2109 (AU)
(74) Representative: Grünecker Patent- und Rechtsanwälte PartG mbB
(86) International application number: PCT/IB2023/050913
(87) International publication number: WO 2023/148649

(56) References cited:
- KR-A- 20160 145 704
- US-A1- 2005 107 845
- US-A1- 2005 107 845
- US-A1- 2008 288 022
- US-A1- 2012 197 065
- US-A1- 2012 197 065
- US-A1- 2017 156 010

## Description

### BACKGROUND

### Field of the Invention

The present invention relates generally to balanced hearing device loudness control.

### Related Art

US 2005/0107845 A1 discloses at least partially fitting a cochlear implant system to a patient by executing a genetic algorithm to select values for a subset of one or more parameters.
US 2012/0197065 A1 discloses systems and methods for displaying and operating a user interface having one or more channel group controllers corresponding to one or more groups of hearing prosthesis channels.
Medical devices have provided a wide range of therapeutic benefits to recipients over recent decades. Medical devices can include internal or implantable components/devices, external or wearable components/devices, or combinations thereof (e.g., a device having an external component communicating with an implantable component). Medical devices, such as traditional hearing aids, partially or fully-implantable hearing prostheses (e.g., bone conduction devices, mechanical stimulators, cochlear implants, *etc.),* pacemakers, defibrillators, functional electrical stimulation devices, and other medical devices, have been successful in performing lifesaving and/or lifestyle enhancement functions and/or recipient monitoring for a number of years.

The types of medical devices and the ranges of functions performed thereby have increased over the years. For example, many medical devices, sometimes referred to as "implantable medical devices," now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, implantable components.

### SUMMARY

One or more non-transitory computer readable storage media according to the invention include the features defined in claim 1.

A system according to the invention includes the features defined in claim 6. .

Embodiments include the features defined in the dependent claims.

### BRIEF DESCRIPTION OF THE DRAWINGS

Embodiments of the present invention are described herein in conjunction with the accompanying drawings, in which:
FIG. 1A is a schematic diagram illustrating a cochlear implant system with which aspects of the techniques presented herein can be implemented;
FIG. 1B is a side view of a recipient wearing a sound processing unit of the cochlear implant system of FIG. 1A;
FIG. 1C is a schematic view of components of the cochlear implant system of FIG. 1A;
FIG. 1D is a block diagram of the cochlear implant system of FIG. 1A;
FIG. 2 is a plot of results of a loudness-scaling test administered to a user of a hearing device with which aspects of the techniques presented herein can be implemented;
FIG. 3A is a plot of a plurality of balanced loudness curves with which aspects of the techniques presented herein can be implemented, and FIG. 3B is a prior art plot of loudness profiles;
FIG. 4 is a plot of probability density functions which indicate probabilities that one or more stimulation levels correspond to one or more perceived loudnesses with which aspects of the techniques presented herein can be implemented;
FIG. 5 is a plot of one or more stimulation levels to one or more perceived loudnesses based on the probability density functions of FIG. 4;
FIGs. 6A, 6B, 6C, 6D, and 6E are respective snapshots of a user interface for viewing and adjusting one or more balanced loudness curves with which aspects of the techniques presented herein can be implemented;
FIGs. 7A, 7B, 7C, 7D, and 7E are respective snapshots of another user interface for viewing and adjusting one or more balanced loudness curves with which aspects of the techniques presented herein can be implemented;
FIG. 8 is a schematic diagram illustrating a computing system with which aspects of the techniques presented herein can be implemented;
FIG. 9 is a flowchart illustrating an example method, in accordance with certain embodiments presented herein;
FIG. 10 is a flowchart illustrating another example method, in accordance with certain embodiments presented herein; and
FIG. 11 is a flowchart illustrating still another example method, in accordance with certain embodiments presented herein.

### DETAILED DESCRIPTION

Today, the volume of a hearing device is typically controlled by adjusting (e.g., increasing or decreasing) the stimulation level for all stimulation channels by a constant (step) current level or a constant (step) percentage of the dynamic range of a given stimulation channel. Even if the master volume is corrected with profile scaling, and even if the user starts with a balanced loudness map (e.g., the same sound at different stimulation channels sound equally loud), existing methods cannot maintain the balanced loudness when the volume is adjusted. This can necessitate an iterative process in which the perceived loudness across stimulation channels undergoes a re-balancing process when the user adjusts the volume of the hearing device.

Therefore, presented herein are techniques to maintain a balanced loudness across stimulation channels when the volume of a hearing device is adjusted. The volume control techniques provided herein may be used to set an overall balanced map loudness and/or to replace existing volume controls in a stand-alone approach. As discussed in greater detail below, the balanced volume control described herein may be used for traditional volume control, self-fitting, acclimatization to loudness, AI-assisted fitting, etc. These techniques presented herein may enable personalized volume control in a user-friendly manner. For example, user loudness data may be collected from a user at home (e.g., via a smartphone) to enable self-fitting.

Merely for ease of description, the techniques presented herein are primarily described with reference to a specific hearing device, namely a cochlear implant. It is to be appreciated that the techniques presented herein may also be partially or fully implemented by other types of hearing devices and/or other types of implantable medical devices. For example, the techniques presented herein may be implemented with middle ear auditory prostheses, bone conduction devices, electro-acoustic prostheses, auditory brain stimulators, direct acoustic stimulations, combinations or variations thereof, *etc.* The techniques presented herein may also be implemented by dedicated tinnitus therapy devices and tinnitus therapy device systems. In further embodiments, the presented herein may also be implemented by, or used in conjunction with, hearables, personal audio devices, in-ear phones, headphones, etc.

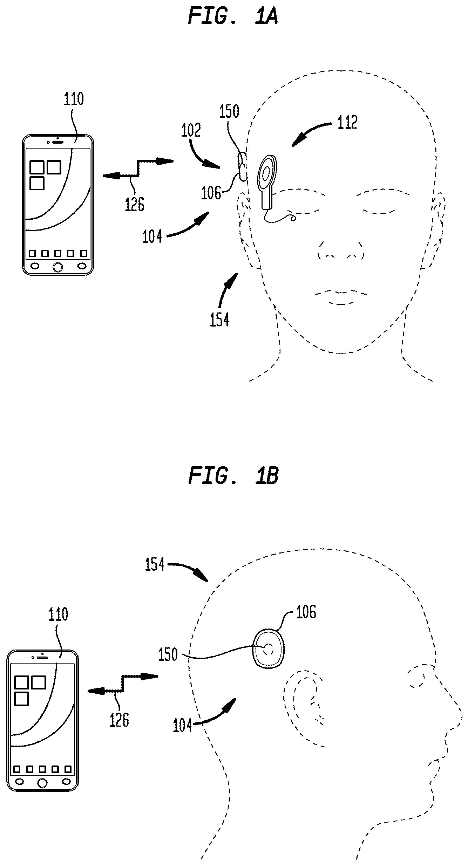

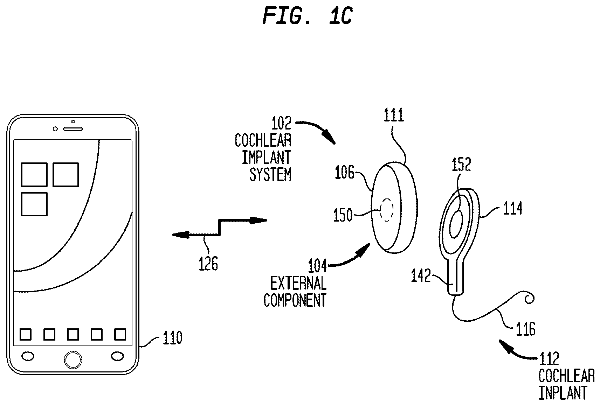

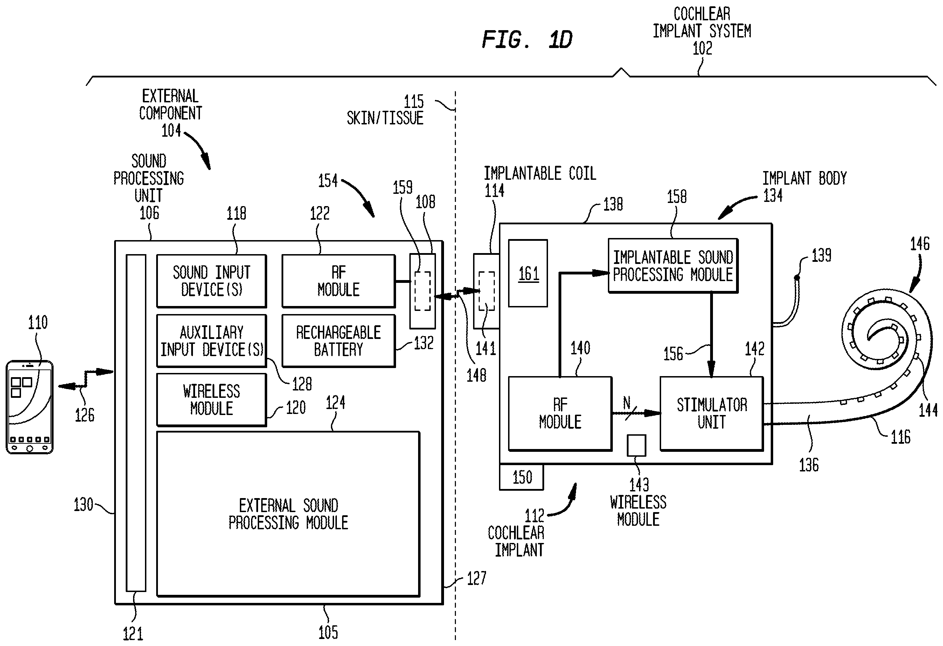

FIGs. 1A-1D illustrate an example cochlear implant system 102 with which aspects of the techniques presented herein can be implemented. The cochlear implant system 102 comprises an external component 104 and an implantable component 112. In the examples of FIGs. 1A-1D, the implantable component is sometimes referred to as a "cochlear implant." FIG. 1A illustrates the cochlear implant 112 implanted in the head 154 of a recipient, while FIG. 1B is a schematic drawing of the external component 104 worn on the head 154 of the recipient. FIG. 1C is another schematic view of the cochlear implant system 102, while FIG. 1D illustrates further details of the cochlear implant system 102. For ease of description, FIGs. 1A-1D will generally be described together.

Cochlear implant system 102 includes an external component 104 that is configured to be directly or indirectly attached to the body of the recipient and an implantable component 112 configured to be implanted in the recipient. In the examples of FIGs. 1A-1D, the external component 104 comprises a sound processing unit 106, while the cochlear implant 112 includes an implantable coil 114, an implant body 134, and an elongate stimulating assembly 116 configured to be implanted in the recipient's cochlea.

In the example of FIGs. 1A-1D, the sound processing unit 106 is an off-the-ear (OTE) sound processing unit, sometimes referred to herein as an OTE component, that is configured to send data and power to the implantable component 112. In general, an OTE sound processing unit is a component having a generally cylindrically shaped housing 111 and which is configured to be magnetically coupled to the recipient's head (e.g., includes an integrated external magnet 159 configured to be magnetically coupled to an implantable magnet 141 in the implantable component 112). The OTE sound processing unit 106 also includes an integrated external (headpiece) coil 108 that is configured to be inductively coupled to the implantable coil 114.

It is to be appreciated that the OTE sound processing unit 106 is merely illustrative of the external devices that could operate with implantable component 112. For example, in alternative examples, the external component may comprise a behind-the-ear (BTE) sound processing unit or a micro-BTE sound processing unit and a separate external. In general, a BTE sound processing unit comprises a housing that is shaped to be worn on the outer ear of the recipient and is connected to the separate external coil assembly via a cable, where the external coil assembly is configured to be magnetically and inductively coupled to the implantable coil 114. It is also to be appreciated that alternative external components could be located in the recipient's ear canal, worn on the body, *etc.*

As noted above, the cochlear implant system 102 includes the sound processing unit 106 and the cochlear implant 112. However, as described further below, the cochlear implant 112 can operate independently from the sound processing unit 106, for at least a period, to stimulate the recipient. For example, the cochlear implant 112 can operate in a first general mode, sometimes referred to as an "external hearing mode," in which the sound processing unit 106 captures sound signals which are then used as the basis for delivering stimulation signals to the recipient. The cochlear implant 112 can also operate in a second general mode, sometimes referred to as an "invisible hearing" mode, in which the sound processing unit 106 is unable to provide sound signals to the cochlear implant 112 (e.g., the sound processing unit 106 is not present, the sound processing unit 106 is powered-off, the sound processing unit 106 is malfunctioning, etc.). As such, in the invisible hearing mode, the cochlear implant 112 captures sound signals itself via implantable sound sensors and then uses those sound signals as the basis for delivering stimulation signals to the recipient. Further details regarding operation of the cochlear implant 112 in the external hearing mode are provided below, followed by details regarding operation of the cochlear implant 112 in the invisible hearing mode. It is to be appreciated that reference to the external hearing mode and the invisible hearing mode is merely illustrative and that the cochlear implant 112 could also operate in alternative modes.

In FIGs. 1A and 1C, the cochlear implant system 102 is shown with an external device 110, configured to implement aspects of the techniques presented. The external device 110 is a computing device, such as a computer (e.g., laptop, desktop, tablet), a mobile phone, remote control unit, *etc.* As described further below, the external device 110 comprises a telephone enhancement module that, as described further below, is configured to implement aspects of the auditory rehabilitation techniques presented herein for independent telephone usage. The external device 110 and the cochlear implant system 102 (e.g., OTE sound processing unit 106 or the cochlear implant 112) wirelessly communicate via a bi-directional communication link 126. The bi-directional communication link 126 may comprise, for example, a short-range communication, such as Bluetooth link, Bluetooth Low Energy (BLE) link, a proprietary link, etc.

Returning to the example of FIGs. 1A-1D, the OTE sound processing unit 106 comprises one or more input devices that are configured to receive input signals (e.g., sound or data signals). The one or more input devices include one or more sound input devices 118 (e.g., one or more external microphones, audio input ports, telecoils, *etc*.), one or more auxiliary input devices 128 (e.g., audio ports, such as a Direct Audio Input (DAI), data ports, such as a Universal Serial Bus (USB) port, cable port, *etc*.), and a wireless transmitter, receiver, and/or transceiver, referred to as a wireless module 120 (e.g., for communication with the external device 110). However, it is to be appreciated that one or more input devices may include additional types of input devices and/or less input devices.

The OTE sound processing unit 106 also comprises the external coil 108, a charging coil 130, a closely-coupled transmitter, receiver, and/or transceiver, referred to as RF module 122, at least one rechargeable battery 132, and an external sound processing module 124. The external sound processing module 124 may comprise, for example, one or more processors and a memory device (memory) that includes sound processing logic. The memory device may comprise any one or more of: Non-Volatile Memory (NVM), Ferroelectric Random Access Memory (FRAM), read only memory (ROM), random access memory (RAM), magnetic disk storage media devices, optical storage media devices, flash memory devices, electrical, optical, or other physical/tangible memory storage devices. The one or more processors are, for example, microprocessors or microcontrollers that execute instructions for the sound processing logic stored in the memory device.

The implantable component 112 comprises an implantable main module (implant body) 134, a lead region 136, and the intracochlear stimulating assembly 116, all configured to be implanted under the skin/tissue (tissue) 115 of the recipient. The implant body 134 generally comprises a hermetically-sealed housing 138 in which an RF module 140 (e.g., an RF receiver, and/or transceiver), a stimulator unit 142, a wireless module 143, an implantable sound processing unit 158, and a rechargeable battery 161 are disposed. The implant body 134 also includes the internal/implantable coil 114 that is generally external to the housing 138, but which is connected to the RF module 140 via a hermetic feedthrough (not shown in FIG. 1D).

As noted, stimulating assembly 116 is configured to be at least partially implanted in the recipient's cochlea. Stimulating assembly 116 includes a plurality of longitudinally spaced intracochlear electrical stimulating contacts (electrodes) 144 that collectively form a contact or electrode array 146 for delivery of electrical stimulation (current) to the recipient's cochlea.

Stimulating assembly 116 extends through an opening in the recipient's cochlea (e.g., cochleostomy, the round window, *etc*.) and has a proximal end connected to stimulator unit 142 via lead region 136 and a hermetic feedthrough (not shown in FIG. 1D). Lead region 136 includes a plurality of conductors (wires) that electrically couple the electrodes 144 to the stimulator unit 142. The implantable component 112 also includes an electrode outside of the cochlea, sometimes referred to as the extra-cochlear electrode (ECE) 139.

As noted, the cochlear implant system 102 includes the external coil 108 and the implantable coil 114. The external magnet 159 is fixed relative to the external coil 108 and the implantable magnet 141 is fixed relative to the implantable coil 114. The magnets fixed relative to the external coil 108 and the implantable coil 114 facilitate the operational alignment of the external coil 108 with the implantable coil 114. This operational alignment of the coils enables the external component 104 to transmit data and power to the implantable component 112 via a closely-coupled wireless link 148 formed between the external coil 108 with the implantable coil 114. In certain examples, the closely-coupled wireless link 148 is a radio frequency (RF) link. However, various other types of energy transfer, such as infrared (IR), electromagnetic, capacitive and inductive transfer, may be used to transfer the power and/or data from an external component to an implantable component and, as such, FIG. 1D illustrates only one example arrangement.

As noted above, sound processing unit 106 includes the external sound processing module 124. The external sound processing module 124 is configured to convert received input signals (received at one or more of the input devices) into output signals for use in stimulating a first ear of a recipient (i.e., the external sound processing module 124 is configured to perform sound processing on input signals received at the sound processing unit 106). Stated differently, the one or more processors in the external sound processing module 124 are configured to execute sound processing logic in memory to convert the received input signals into output signals that represent electrical stimulation for delivery to the recipient.

As noted, FIG. 1D illustrates an embodiment in which the external sound processing module 124 in the sound processing unit 106 generates the output signals. In an alternative embodiment, the sound processing unit 106 can send less processed information (e.g., audio data) to the implantable component 112 and the sound processing operations (e.g., conversion of sounds to output signals) can be performed by a processor within the implantable component 112.

Returning to the specific example of FIG. 1D, the output signals are provided to the RF module 122, which transcutaneously transfers the output signals (e.g., in an encoded manner) to the implantable component 112 via external coil 108 and implantable coil 114. That is, the output signals are received at the RF module 140 via implantable coil 114 and provided to the stimulator unit 142. The stimulator unit 142 is configured to utilize the output signals to generate electrical stimulation signals (e.g., current signals) for delivery to the recipient's cochlea. In this way, cochlear implant system 102 electrically stimulates the recipient's auditory nerve cells, bypassing absent or defective hair cells that normally transduce acoustic vibrations into neural activity, in a manner that causes the recipient to perceive one or more components of the received sound signals.

As detailed above, in the external hearing mode the cochlear implant 112 receives processed sound signals from the sound processing unit 106. However, in the invisible hearing mode, the cochlear implant 112 is configured to capture and process sound signals for use in electrically stimulating the recipient's auditory nerve cells. In particular, the cochlear implant 112 includes at least an implantable sound sensor arrangement 150 comprising one or more implantable sound sensors (e.g., an implantable microphone and/or an implantable accelerometer).

Similar to the external sound processing module 124, the implantable sound processing module 158 may comprise, for example, one or more processors and a memory device (memory) that includes sound processing logic. The memory device may comprise any one or more of: Non-Volatile Memory (NVM), Ferroelectric Random Access Memory (FRAM), read only memory (ROM), random access memory (RAM), magnetic disk storage media devices, optical storage media devices, flash memory devices, electrical, optical, or other physical/tangible memory storage devices. The one or more processors are, for example, microprocessors or microcontrollers that execute instructions for the sound processing logic stored in memory device.

In the invisible hearing mode, the implantable sound sensor 150, potentially in cooperation with one or more other implantable sensors, such as an implantable vibration sensor (not shown in FIGs. 1A-1D), is configured to detect/capture signals (e.g., acoustic sound signals, vibrations, etc.), which are provided to the implantable sound processing module 158. The implantable sound processing module 158 is configured to convert received input signals (received at the implantable sound sensor 150) into electrical signals, sometimes referred to herein as sensed, received, or captured sound signals, for use in stimulating the first ear of a recipient (i.e., the processing module 158 is configured to perform sound processing operations). Stated differently, the one or more processors in implantable sound processing module 158 are configured to execute sound processing logic in memory to convert the received sound signals into output signals 156 that are provided to the stimulator unit 142. The stimulator unit 142 is configured to utilize the output signals 156 to generate electrical stimulation signals (e.g., current signals) for delivery to the recipient's cochlea, thereby bypassing the absent or defective hair cells that normally transduce acoustic vibrations into neural activity.

It is to be appreciated that the above description of the so-called external hearing mode and the so-called invisible hearing mode are merely illustrative and that the cochlear implant system 102 could operate differently in different embodiments. For example, in one alternative implementation of the external hearing mode, the cochlear implant 112 could use signals captured by the sound input devices 118 and the implantable sound sensor 150 in generating stimulation signals for delivery to the recipient. In other embodiments, the cochlear implant 112 could operate substantially or completely without the external component 104. That is, in such embodiments, the cochlear implant 112 could operate substantially or completely in the invisible hearing mode using the rechargeable battery 161. The rechargeable battery 161 would be recharged via an external charging device.

After a user first receives a hearing device, the user is typically polled for feedback regarding the perceived loudness (also referred to herein as "perceived auditory intensity level") of various stimulation signals at multiple stimulation channels (e.g., frequency bands/electrodes in electrical stimulation devices). This process may be referred to as "fitting" or "mapping" of the device. Based on the feedback, a comfort level (C-level) and/or threshold level (T-level) can be determined. The C-level and the T-level are, respectively, the maximum and minimum loudness levels that are acceptable to the user at a given stimulation channels. For example, the user may be uncomfortable with any stimulation signal that is greater than the C-level for a given stimulation channel; conversely, the user may have difficulty hearing any stimulation signal that is smaller than the T-level of a given stimulation channel. When delivered to the user, stimulation signals are generally "mapped" to some value/level between the T-level and the C-level depending on the actual sound energy present at that time.

Today, the loudness of a hearing device can be adjusted by a clinician (e.g., using level shifting in fitting software) or the user (e.g., using volume control). The stimulation levels are adjusted (e.g., increased/decreased) by the same fixed step across all stimulation channels or the stimulation levels are adjusted by the fixed percentage of the dynamic range across all stimulation channels. With such changes, the "perceived" loudness can become unbalanced across stimulation channels during stimulation level adjustments, even if the stimulation levels are initially balanced for perceived loudness across stimulation channels. For example, consider a fixed step or percentage stimulation level adjustment that is implemented for all stimulation channels. The resulting perceived loudness at a first stimulation channel might have been raised or lowered more than the resulting perceived loudness at a second stimulation channel. That is, even though every stimulation channel experiences the same fixed step or percentage adjustment to the stimulation level, the loudness perceived by the user across the stimulation channels can become unbalanced.

The reason that perceived loudness can become unbalanced is that neither existing option (fixed step or fixed percentage) can maintain the relationship of the perceived loudness between channels. The fixed step option fails because the dynamic ranges vary across stimulation channels. As a result, a fixed step may be relatively small on one stimulation channel but relatively large on another. Meanwhile, a fixed percentage option fails because the relationship between stimulation level and perceived loudness is not necessarily linear. Thus, existing techniques cannot adequately maintain the balance of perceived loudness across stimulation channels when the stimulation levels are adjusted.

Accordingly, techniques are presented herein for a personalized volume control that maintains the same adjustment to perceived loudness across all stimulation channels. In one example, one or more "balanced loudness curves" (or "balanced auditory intensity curves") can be used to maintain the relationship of perceived loudness across stimulation channels. Each balanced loudness curve may include a set of stimulation levels (or "stimulus intensities," "electrical stimulus levels," etc.) for respective stimulation channels, with each set representing a specific perceived loudness across stimulation channels. By switching between different balanced loudness curves - instead of raising or lowering stimulation levels based on a fixed step or fixed percentage - the hearing device may adjust the perceived loudness in a balanced/constant manner across stimulation channels.

In certain embodiments, the balanced loudness curves can be constructed based on data obtained from a loudness-scaling test administered to a user of a hearing device. The loudness-scaling test can probe the perceived loudness at different stimulation levels and stimulation channels by randomly stimulating an electrode and prompting the user to rate the corresponding perceived loudness on a categorical scale. The categorical scale may, for example, include a range from L0-L50, where L0 means that the user cannot hear the stimulation signal and L50 means that the stimulation signal is too loud. In one example, the loudness scaling test may be performed for three adjacent electrodes simultaneously; however, it will be appreciated that any suitable number of electrodes may be tested in the loudness scaling test simultaneously and/or sequentially. The loudness scaling test may be administered at any suitable location (e.g., home) via a mobile device of the user. Furthermore, the loudness scaling test may involve any suitable stimulus, such as speech-like modulations.

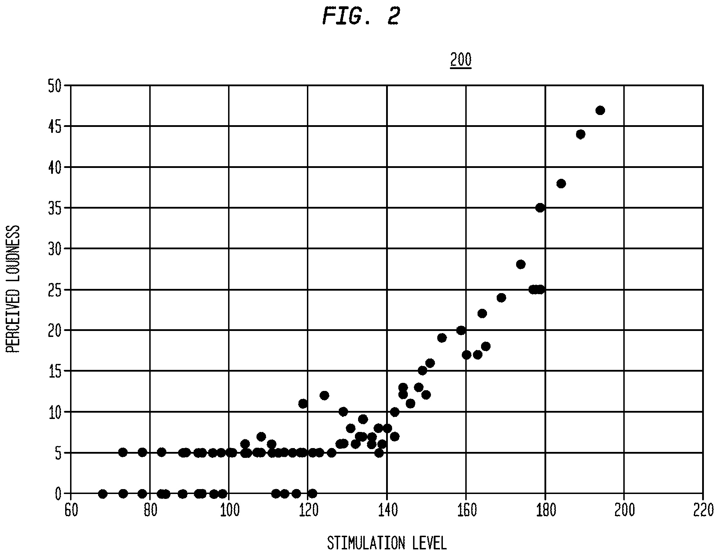

FIG. 2 is a plot 200 of results of one or more loudness-scaling tests (e.g., Electrical Loudness Scaling (ELS) tests) administered to a user of a hearing device. Plot 200 represents the results for a single stimulation channel. The horizontal axis represents the stimulation level, and the vertical axis represents the perceived loudness on a scale from 0-50. In this example, as shown, the perceived loudness increases with the stimulation level. The loudness scaling test(s) may collect multiple data points (perceived loudness indications) for each stimulation level in the stimulation channel.

The loudness-scaling test(s) that generated plot 200 may also be used to produce additional plots for other stimulation channels. Thus, for each of a plurality of stimulation channels of the hearing device, one or more indications may be obtained of perceived loudnesses of a plurality of stimulation signals delivered to the user via a corresponding stimulation channel/electrode(s). Based on the data shown in plot 200 and any other plots, a plurality of balanced loudness curves for the user may be determined.

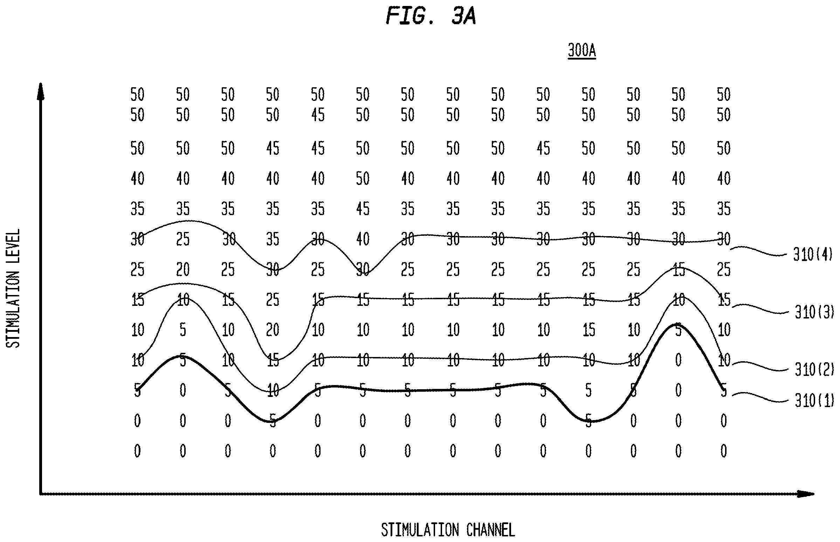

The ELS test(s) performed at multiple electrodes may provide data for an initial tuning of a loudness mapping (e.g., plot 200) from which one or more balanced loudness curves may be derived. The balanced loudness curves may include a set of mappings that maintain the loudness relation (e.g., keep the balance) across stimulation channels for a particular user. For example, FIG. 3A is a plot 300A of balanced loudness curves 310(1)-310(4). The horizontal axis represents stimulation channels, and the vertical axis represents stimulation level. For each [stimulation channel, stimulation level] pairing, plot 300A shows the perceived loudness on a scale from 0-50.

Balanced loudness curves 310(1)-310(4) relate the stimulation level associated with a given perceived loudness, for each stimulation channel. In particular, balanced loudness curves 310(1)-310(4) represent the stimulation levels, for each respective channel, which elicit a perceived loudness of 5, 10, 15, and 30, respectively. Thus, balanced loudness curves 310(1)-310(4) may be a set of stimulation levels that generate the same perceived loudness across stimulation channels.

Conventionally, a loudness curve/profile can be determined during a general fitting session in which a clinician balances the individual electrodes at settings which are loud but comfortable. However, because broadband loudness is unpredictably related to (but generally considerably higher than) single-channel loudness, the complete map across all channels may be too loud to switch on. This is why the loudness data collected as part of a conventional fitting session cannot be directly used to set the correct loudness level. Instead, conventionally, all levels would be dropped (e.g., by a fixed step, such as by 10 perceived loudness levels, or by a fixed percentage of the dynamic range of each channel, such as 20%), resulting in an unbalanced loudness curve. Then, the clinician would switch on the processor and adjust the loudness setting up or down, generating further unbalanced loudness curves. Thus, conventional volume control techniques create loudness profiles that could be unbalanced over different stimulation channels, even if the volume control process begins with a balanced profile.

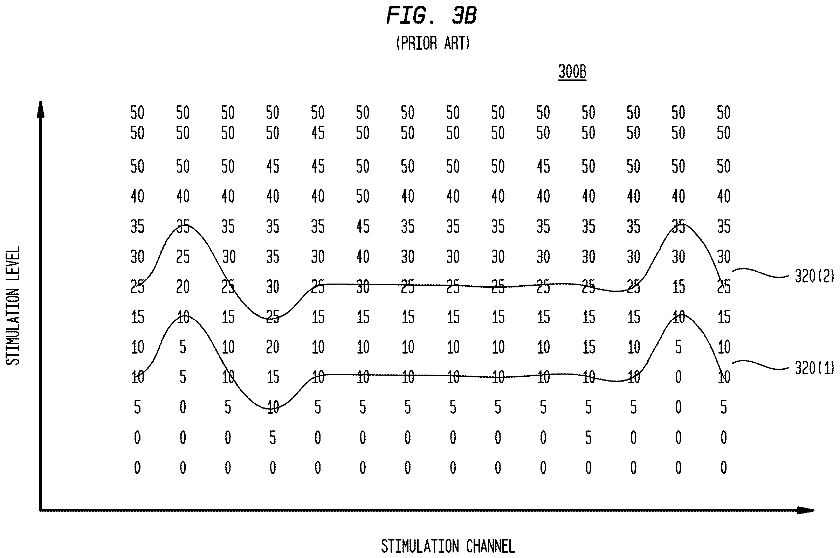

This principle is demonstrated in FIG. 3B, which illustrates a prior art plot 300B of loudness profiles 320(1) and 320(2). Loudness profiles 320(1) and 320(2) maintain the same relative stimulation level, but have different perceived loudnesses, across stimulation channels. As shown, loudness profile 320(1) is initially balanced at perceived loudness 10. The conventional volume control process continues by adjusting loudness profile 320(1) by a fixed step, as described above. In this example, a user may shift loudness profile 320(1) upward, generating loudness profile 320(2). Loudness profile 320(2) is unbalanced because it includes perceived loudnesses of 25, 30, and 35 at various stimulation channels.

Unlike loudness profiles 320(1) and 320(2), balanced loudness curves 310(1)-310(4) (FIG. 3A) maintain balanced perceived loudnesses across stimulation channels. As a result, instead of using a fixed step or percentage, the user may cycle through consecutive balanced loudness curves 310(1)-310(4), thereby enabling personalized volume control on a per-user basis which maintains loudness balance over the array of stimulation channels. Balanced loudness curves 310(1)-310(4) may thus enable a user to control a volume level of a hearing device while maintaining the same perceived loudness across all stimulation channels.

In one example, the hearing device may be programmed with balanced loudness curves 310(1)-310(4). Balanced loudness curves 310(1)-310(4) may be selectable by the user to control a loudness of stimulation signals delivered to the user. Thus, a selection (e.g., a user selection) may be obtained of one of balanced loudness curves 310(1)-310(4). The selection may be obtained as part of an initial calibration or every-day use. In the case of initial calibration, balanced loudness curves 310(1)-310(4) may be used to convert/translate test audio signals to test stimulation signals, and in response, a selection of one of balanced loudness curves 310(1)-310(4) may be obtained. In the case of every-day use, a user indication to change from one of balanced loudness curves 310(1)-310(4) to another one of balanced loudness curves 310(1)-310(4) may be obtained, and the other one of balanced loudness curves 310(1)-310(4) may be used to translate audio signals to stimulation signals. In any event, audio signals may be received at the hearing device and converted/translated to stimulation signals using the selected one of balanced loudness curves 310(1)-310(4). Once converted, the stimulations signals may be delivered to the user. The stimulation signals may include mechanical, electrical, or acoustic stimulation signals, depending on the particular type of hearing device.

In one example, an indication may be obtained to switch to another one of balanced loudness curves 310(1)-310(4). For example, if balanced loudness curve 310(4) is initially selected, but the user then enters a noisy environment, the user may provide an indication to switch to balanced loudness curve 310(1). Or, if balanced loudness curve 310(1) is initially selected, but the user enters a quiet environment, the user provide an indication to switch to balanced loudness curve 310(4).

Balanced loudness curves 310(1)-310(4) may represent consecutive profiles/mappings of any suitable density. Each of balanced loudness curves 310(1)-310(4) may be louder or softer than other ones of balanced loudness curves 310(1)-310(4), while maintaining the loudness relations between stimulation channels.

It will be appreciated that the techniques described herein may use any suitable number of balanced loudness curves corresponding to any suitable number of stimulation levels. The balanced loudness curves may extend over any suitable number of stimulation channels. Furthermore, the balanced loudness curves may be any suitable shape. That is, the balanced loudness curves may relate any suitable stimulation levels to any suitable stimulation channels such that the respective stimulation levels correspond to the same perceived loudness across all stimulation channels.

Techniques described herein may also apply to users with multiple hearing devices. For example, consider a user with bilateral devices, one on each side of the user's head. In this case, the balanced loudness curves may extend across stimulation channels on both hearing devices. Thus, a single balanced loudness control may be provided for the two hearing devices. For instance, when a user opts to adjust the perceived volume of the hearing devices, both hearing devices may adjust the stimulation levels for each stimulation channel according to the relevant balanced loudness curve(s).

Controlling multiple devices as described herein may be referred to as "broadband" control. In one example, individual balanced loudness curves for each hearing device may be determined based on one or more loudness-scaling tests (e.g., ELS tests). After the individual balanced loudness curves are separately determined for the two hearing devices, a broadband loudness-scaling test may be administered to the user of the hearing devices. The broadband loudness-scaling test may measure/compare the perceived loudnesses relative to each other (e.g., how loud each balanced loudness curve sounds to the user). Based on the broadband loudness-scaling test, the hearing devices may be synchronized. For example, the individual balanced loudness curves may be mapped to each other across the hearing devices to enable singular personalized volume control of both hearing devices.

The relationship between stimulation level and loudness may not necessarily be deterministic - that is, if a stimulation level is repeatedly administered, the user may rate that stimulation level with different perceived loudnesses. For instance, earlier sounds can influence the perceived loudness rated by a user. Therefore, instead of a fixed/deterministic mapping, certain examples may use a probabilistic model to generate balanced loudness curves. For example, instead of a deterministic model that indicates that a stimulation level of 160 results in a perceived loudness of L25, a probabilistic model might indicate that a stimulation level of 160 has a 5% probability of resulting in a perceived loudness of L20, an 80% probability of resulting in a perceived loudness of L25, and a 15% probability of resulting in a perceived loudness of L30. One example of a probabilistic model that may be used in conjunction with the techniques described herein is discussed in Trevino et al., "Development of a Multi-Category Psychometric Function to Model Categorical Loudness Measurements," J. Acoust. Soc. Am. 140 (4), October 2016.

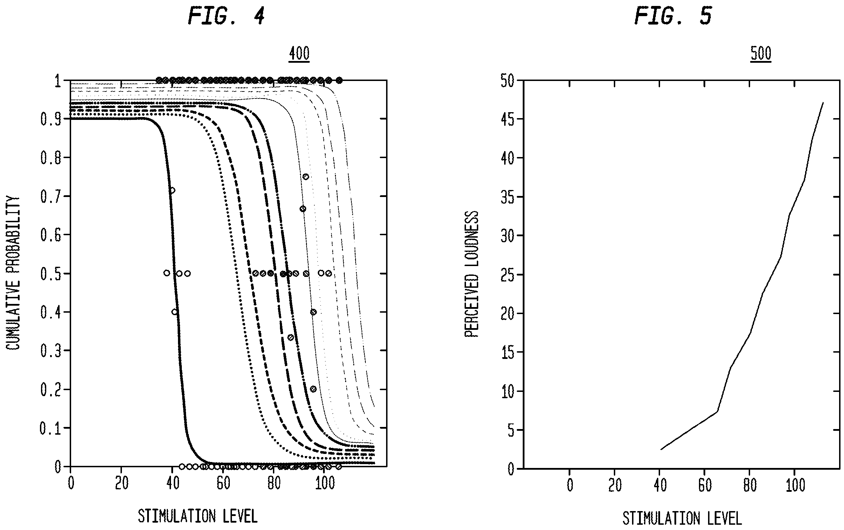

One or more balanced loudness curves may be constructed based on one or more probability density functions which indicate probabilities that one or more stimulation levels correspond to one or more perceived loudnesses. FIG. 4 is a plot 400 of such probability density functions. The horizontal axis represents the stimulation level, and the vertical axis represents the cumulative probability. The curves shown in plot 400 are probability density functions that correspond to respective perceived loudnesses. Thus, a given probability density function shown in plot 400 represents a distribution of probabilities that a given stimulation level corresponds to a given perceived loudness. Plot 400 may correspond to a given stimulation channel, and multiple other plots many be generated for other stimulation channels.

The probability density functions may be generated using any suitable/available data. For example, the probability density functions may be pre-fed with *a priori* estimates based on population averages and updated in a Bayesian process as loudness scaling data becomes available. The probability density functions may be based on large data sets obtained from ELS tests of many users.

One or more balanced loudness curves may be constructed using any suitable mechanism, such as an Artificial Intelligence (AI) process (e.g., a Machine Learning (ML) process) or a rule-based approach. In one example, the probability density functions shown in plot 400 may be generated by using AI/ML processes to statistically fit the data. For example, FIG. 5 is a plot 500 of one or more stimulation levels to one or more perceived loudnesses based on the probability density functions of FIG. 4. The horizontal axis represents the stimulation level, and the vertical axis represents the perceived loudness. The curve shown in plot 500 may be a best-fit curve generated by the AI/ML processes based on the data shown in plot 400. In one example, the loudness control may be expressed in equal amounts of probability change across stimulation channels.

In a further example, an AI/ML process may be used to identify different collections of probability density functions and/or balanced loudness curves. For instance, different group of users may rate respective perceived loudness for respective stimulation levels. The AI/ML process may assign a given user to one of the identified groups depending on the results of the user's loudness scaling data. Thus, a given probabilistic loudness model may be built and fit to the user. The resulting probabilistic loudness map/model may be used to create an individualized volume control that maintains balanced loudness relationships between stimulation channels based on one or more best-fit balanced loudness curves. The volume control may be used in a Master Volume, Bass, and Treble (MVBT) - like fitting.

Thus, in one example, ELS test data may be obtained from a plurality of users and used to generate one or more probability density functions (e.g., as shown in plot 400). Based on the one or more probability density functions, a plurality of balanced loudness curves may be constructed for a given user. For instance, multiple probability density functions may be generated and used to construct multiple probabilistic balanced loudness curves (e.g., balanced loudness curves based on historical ELS test data). The balanced loudness curves for the given user may then be manually or automatically selected from among the probabilistic balanced loudness curves.

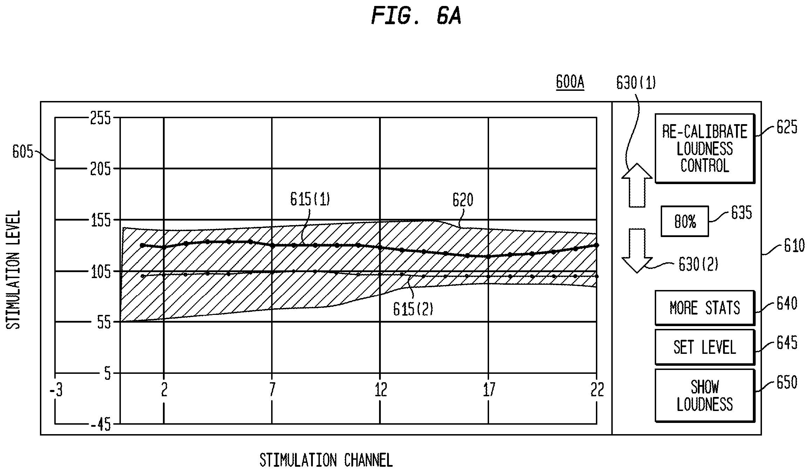

FIGs. 6A-6E are respective snapshots 600A-600E of a user interface for viewing/adjusting/fitting one or more balanced loudness curves. Briefly, the user interface may enable a user to tune/control perceived volume for one or more hearing devices. In one example, the user interface may be used for an initial calibration/fitting of balanced loudness curves for a user. In another example, the user interface may be used for post-calibration, active volume control.

With reference first to FIG. 6A, snapshot 600A includes plot display 605 and control panel 610. The horizontal axis of plot display 605 represents the stimulation channel, and the vertical axis is displayed in units of the stimulation level. Plot display 605 shows balanced loudness curves 615(1) and 615(2) and overlay 620. Overlay 620 represents a percentage of the normal boundary for loudness levels based on population data.

Control panel 610 includes re-calibration button 625, arrows 630(1) and 630(2), percentage indicator 635, statistics button 640, set level button 645, and show loudness button 650. Re-calibration button 625, when selected, prompts calibration of loudness increases using the loudness scale (personalized volume control). Arrows 630(1) and 630(2), when selected, prompt adjustment of the volume. Percentage indicator 635 displays an indication of the stimulation level as a percentage (e.g., 80%) of the population (e.g., as shown in overlay 620). Statistics button 640, when selected, may prompt display of further statistics associated with the user's personalized volume control data. Set level button 645, when selected, enables the user to manually control loudness levels in case adjustments are needed. Show loudness button 650, when selected, prompts display of additional loudness data. In the example of FIG. 6A, show loudness button 650 has not yet been selected.

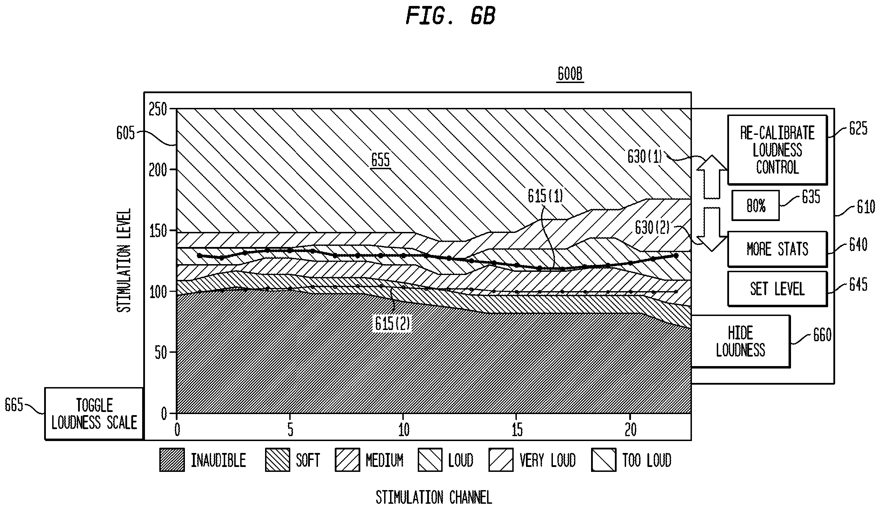

FIG. 6B is a snapshot 600B of the user interface when show loudness button 650 is selected. As shown, snapshot 600B includes overlay 655 which represents the loudness estimates obtained from loudness data that has been collected and processed by a probabilistic model (e.g., a model described above in connection with FIGs. 4 and 5). Thus, overlay 655 may display a plurality of probabilistic balanced loudness curves constructed from a plurality of probability density functions. Overlay 655 is presented in the form of a superimposed gradient, with each section of the gradient representing a different level/category of perceived loudness measurements. The categories may include inaudible, soft, medium, loud, very loud, and too loud. Overlay 655 may enable a user to visually determine how balanced loudness curves 615(1) and 615(2) - which may be based on individual user-reported data - differ from the probabilistic loudness data, which may be based on historical data reported by many users. In one example, the user may adjust balanced loudness curves 615(1) and 615(2) to more closely match the probabilistic loudness data, if desired.

In this example, show loudness button 650 has been replaced with hide loudness button 660 which, when selected, removes overlay 655 and returns the user interface to snapshot 600A. In addition, toggle loudness scale button 665 is now present. When selected, toggle loudness scale button 665 may prompt the vertical axis of plot display 605 to toggle between stimulation level and perceived volume of historical data.

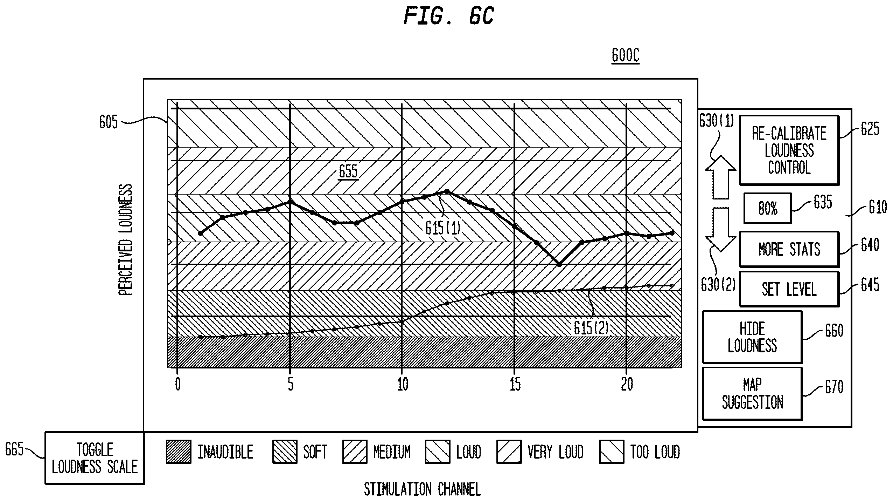

FIG. 6C is a snapshot 600C of the user interface when toggle loudness scale button 665 is selected. Here, the vertical axis is now scaled in linear units of perceived loudness instead of stimulation level. Thus, the same data is displayed (e.g., balanced loudness curves 615(1) and 615(2) and overlay 655), albeit in a re-scaled format. In particular, balanced loudness curves 615(1) and 615(2) are distorted relative to snapshots 600A and 600B, and each section of the gradient in overlay 655 is separated by a straight line across the horizontal axis. That is, in snapshot 600C, the probabilistic balanced loudness curves may be shown as horizontal lines. In addition, map suggestion button 670 is now present. When selected, map suggestion button 670 may prompt a display of one or more suggested balanced loudness curves.

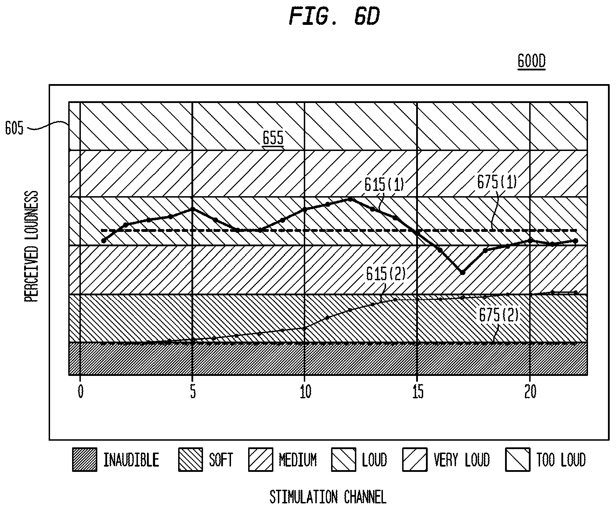

FIG. 6D is a snapshot 600D of the user interface when map suggestion button 670 is selected. **In** this example, the vertical axis of plot display 605 continues to be scaled in units of perceived loudness, and balanced loudness curves 615(1) and 615(2) and overlay 655 are shown. However, suggested balanced loudness curves 675(1) and 675(2) are now also displayed. Suggested balanced loudness curves 675(1) and 675(2) may be based on historical data from a large number of users. In this parameter space, suggested balanced loudness curves 675(1) and 675(2) may be straight, parallel lines. Vertical displacement of these parallel lines may enable balanced volume control.

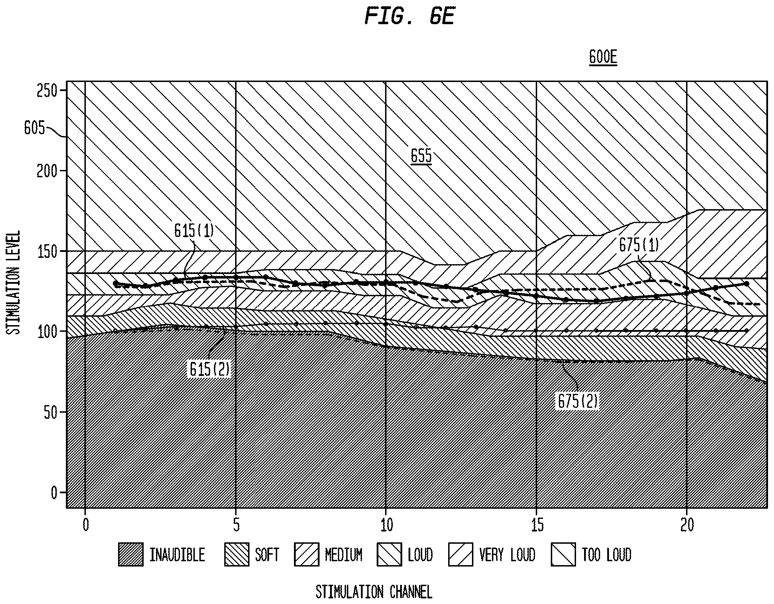

FIG. 6E is a snapshot 600E of the user interface when map suggestion button 670 is selected. In this example, the vertical axis of plot display 605 is now scaled in terms of stimulation level. As a result, balanced loudness curves 615(1) and 615(2) and overlay 655 take on the same profiles as shown in snapshot 600B (FIG. 6B). Furthermore, because the parameter space has changed, suggested balanced loudness curves 675(1) and 675(2) are no longer straight or parallel.

Arrows 630(1) and 630(2) may provide similar functionality for both calibration mode and active/every-day use mode. In either mode, arrows 630(1) and 630(2) may serve as toggle buttons for selecting one of balanced loudness curves 615(1) and 615(2). When a selection of one of balanced loudness curves 615(1) and 615(2) is obtained via the arrows 630(1) and 630(2), the selected balanced loudness curve may be used to translate audio signals to stimulation signals. As illustrated in snapshots 600A-600E, volume control may be expressed in steps of perceived loudness or stimulation level. In the former case, for example, selecting arrow 630(1) five times may cause the perceived loudness to increase by five units (e.g., from L10 to L15). In the latter case, for example, selecting arrow 630(1) may prompt a maximum stimulation level change in any stimulation channel (e.g., the maximum stimulation level change may be three units).

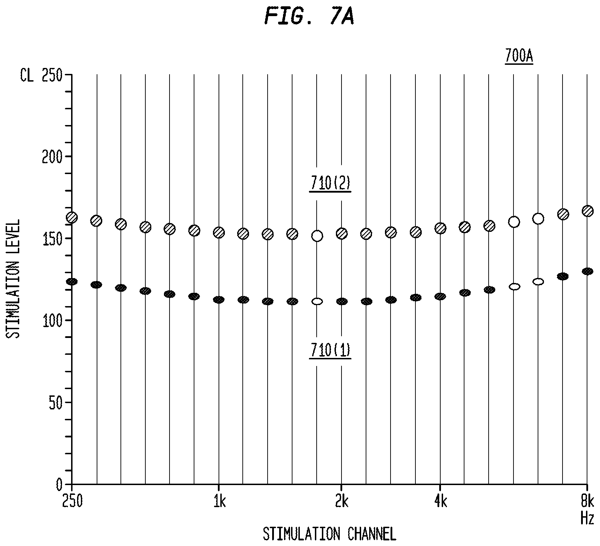

FIGs. 7A-7E are respective snapshots 700A-700E of another user interface for viewing and adjusting one or more balanced loudness curves. The user interface may function as a suggestion screen for a user's balanced loudness curves. With reference first to FIG. 7A, snapshot 700A includes a plot with a horizontal axis representing the stimulation channel, and a vertical axis representing the stimulation level. The plot shows balanced loudness curves 710(1) and 710(2) based on individual, user-reported loudness data (e.g., from an ELS test).

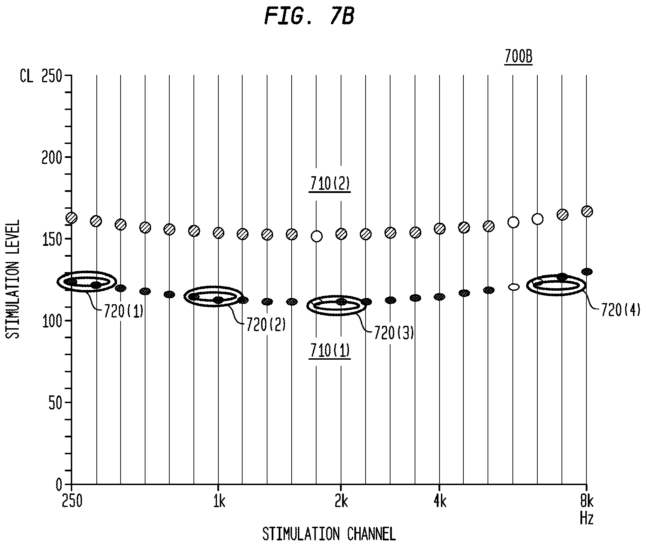

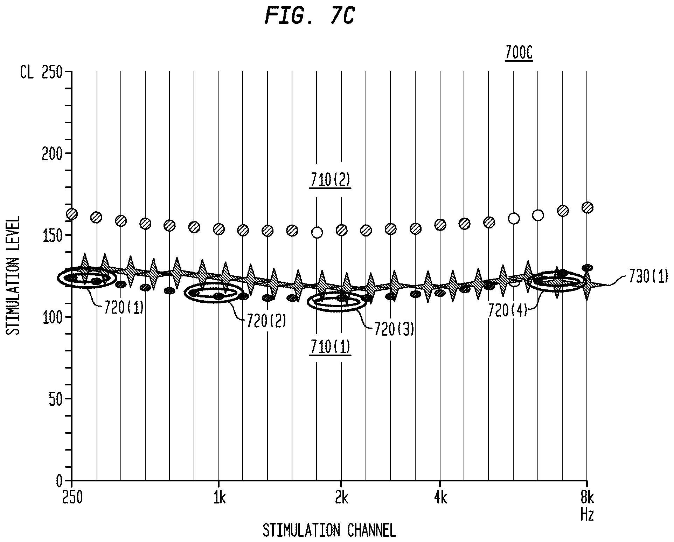

FIG. 7B is a snapshot 700B that illustrates balanced loudness curves 710(1) and 710(2) as well indicators 720(1)-720(4). Indicators 720(1)-720(4), which may be rated as "very soft," may illustrate the boundary below which stimulation levels are difficult to hear. As shown in snapshot 700C (FIG. 7C), a model (e.g., an AI/ML process) may analyze data corresponding to indicators 720(1)-720(4) to arrive at suggested balanced loudness curve 730(1). In one example, a user may select suggested balanced loudness curve 730(1) for use instead of balanced loudness curve 710(1).

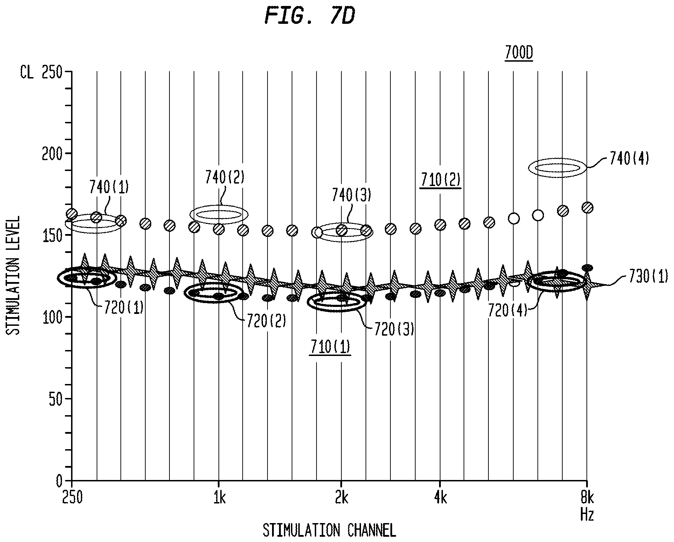

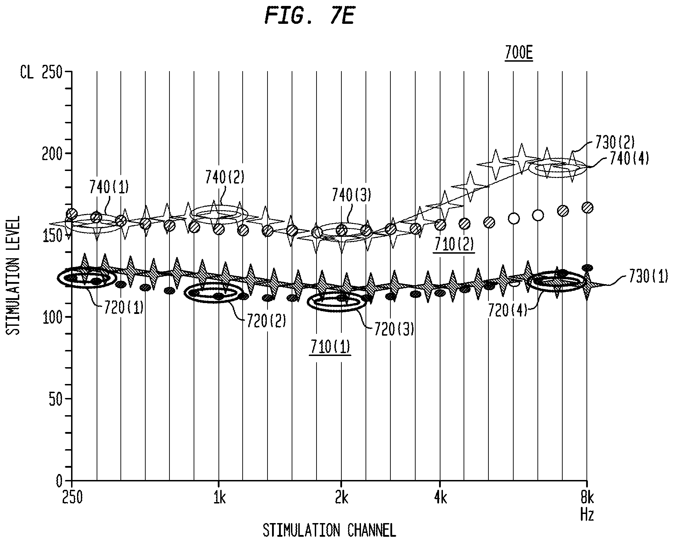

FIG. 7D is a snapshot 700D that illustrates balanced loudness curves 710(1) and 710(2), indicators 720(1)-720(4), and suggested balanced loudness curve 730(1), as well as indicators 740(1)-740(4). Indicators 740(1)-740(4), which may be rated as "loud," may illustrate the boundary above which stimulation levels are uncomfortably loud. As shown in snapshot 700E (FIG. 7E), a model (e.g., an AI/ML process) may analyze data corresponding to indicators 740(1)-740(4) to arrive at suggested balanced loudness curve 730(2). In one example, a user may select suggested balanced loudness curve 730(2) for use instead of balanced loudness curve 710(2).

As described herein, a personalized loudness control may be built based on loudness scaling and may be used to set the overall volume of the hearing device to a desired level. One specific example process may involve (1) for each stimulation channel, building a loudness map relating perceived loudness to stimulation level; (2) building a loudness profile for a given stimulation level; (3) providing a personalized balanced volume control; (4) turning down the map's loudness using the personalized volume control; and (5) turning-up the personalized volume control to a comfortable level.

The technology disclosed herein can be applied in any of a variety of circumstances and with a variety of different devices. Aspects of the techniques presented may make use of a computing device (e.g., example cochlear implant system 102 illustrated in FIGs. 1A-1D). Such computing devices may benefit from technology disclosed herein.

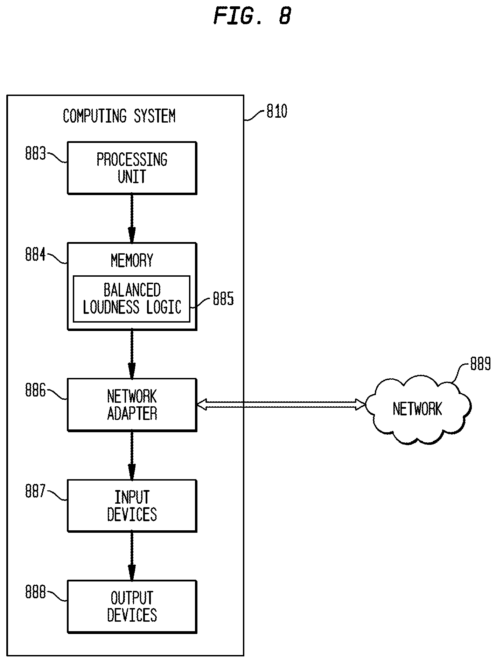

FIG. 8 illustrates an example of a suitable computing system 810 with which one or more of the disclosed examples can be implemented. Computing systems, environments, or configurations that can be suitable for use with examples described herein include, but are not limited to, personal computers, server computers, hand-held devices, laptop devices, multiprocessor systems, microprocessor-based systems, programmable consumer electronics (e.g., smart phones), network PCs, minicomputers, mainframe computers, tablets, distributed computing environments that include any of the above systems or devices, and the like. The computing system 810 can be a single virtual or physical device operating in a networked environment over communication links to one or more remote devices, such as an implantable medical device or implantable medical device system.

In its most basic configuration, computing system 810 includes at least one processing unit 883 and memory 884. The processing unit 883 includes one or more hardware or software processors (e.g., Central Processing Units) that can obtain and execute instructions. The processing unit 883 can communicate with and control the performance of other components of the computing system 810.

The memory 884 is one or more software or hardware-based computer-readable storage media operable to store information accessible by the processing unit 883. The memory 884 can store, among other things, instructions executable by the processing unit 883 to implement applications or cause performance of operations described herein, as well as other data. The memory 884 can be volatile memory (e.g., RAM), non-volatile memory (e.g., ROM), or combinations thereof. The memory 884 can include transitory memory or non-transitory memory. The memory 884 can also include one or more removable or non-removable storage devices. In examples, the memory 884 can include RAM, ROM, EEPROM (Electronically-Erasable Programmable Read-Only Memory), flash memory, optical disc storage, magnetic storage, solid state storage, or any other memory media usable to store information for later access. In examples, the memory 884 encompasses a modulated data signal (e.g., a signal that has one or more of its characteristics set or changed in such a manner as to encode information in the signal), such as a carrier wave or other transport mechanism and includes any information delivery media. By way of example, and not limitation, the memory 884 can include wired media such as a wired network or direct-wired connection, and wireless media such as acoustic, RF, infrared and other wireless media or combinations thereof. In certain embodiments, the memory 884 comprises balanced loudness logic 885 that, when executed, enables the processing unit 883 to perform aspects of the techniques presented.

In the illustrated example, the system 810 further includes a network adapter 886, one or more input devices 887, and one or more output devices 888. The system 810 can include other components, such as a system bus, component interfaces, a graphics system, a power source (e.g., a battery), among other components.

The network adapter 886 is a component of the computing system 810 that provides network access (e.g., access to at least one network 889). The network adapter 886 can provide wired or wireless network access and can support one or more of a variety of communication technologies and protocols, such as ETHERNET, cellular, BLUETOOTH, near-field communication, and RF (Radio Frequency), among others. The network adapter 886 can include one or more antennas and associated components configured for wireless communication according to one or more wireless communication technologies and protocols.

The one or more input devices 887 are devices over which the computing system 810 receives input from a user. The one or more input devices 887 can include physically-actuatable user-interface elements (e.g., buttons, switches, or dials), touch screens, keyboards, mice, pens, and voice input devices, among others input devices.

The one or more output devices 888 are devices by which the computing system 810 is able to provide output to a user. The output devices 888 can include, displays, speakers, and printers, among other output devices.

It is to be appreciated that the arrangement for computing system 810 shown in FIG. 8 is merely illustrative and that aspects of the techniques presented herein may be implemented at a number of different types of systems/devices. For example, the computing system 810 could be a laptop computer, tablet computer, mobile phone, surgical system, etc.

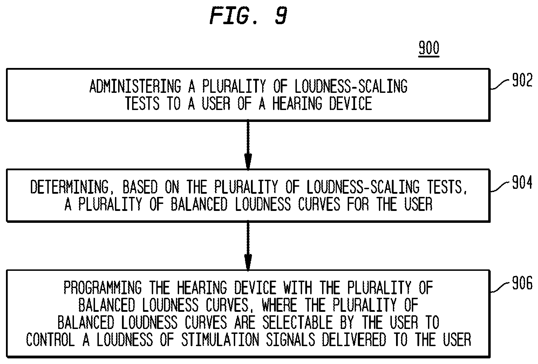

FIG. 9 is a flowchart of an example method 900, in accordance with embodiments presented herein. Method 900 begins at 902 where a plurality of loudness-scaling tests are administered to a user of a hearing device. At 904, based on the plurality of loudness-scaling tests, a plurality of balanced loudness curves are determined for the user. At 906, the hearing device is programmed with the plurality of balanced loudness curves, where the plurality of balanced loudness curves are selectable by the user to control a loudness of stimulation signals delivered to the user.

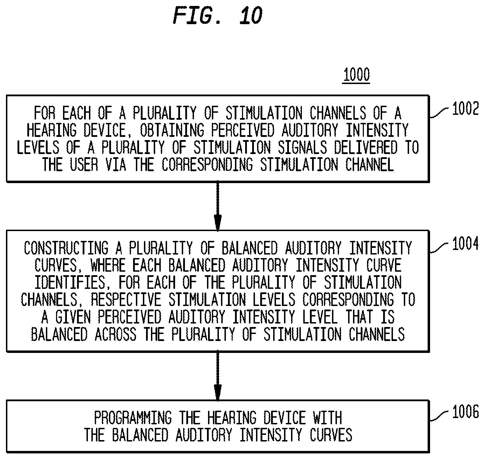

FIG. 10 is a flowchart of an example method 1000, in accordance with embodiments presented herein. Method 1000 begins at 1002 where, for each of a plurality of stimulation channels of a hearing device, perceived auditory intensity levels of a plurality of stimulation signals delivered to the user via the corresponding stimulation channel are obtained. At 1004, a plurality of balanced auditory intensity curves is constructed, where each balanced auditory intensity curve identifies, for each of the plurality of stimulation channels, respective stimulation levels corresponding to a given perceived auditory intensity level that is balanced across the plurality of stimulation channels. At 1006, the hearing device is programmed with the balanced auditory intensity curves.

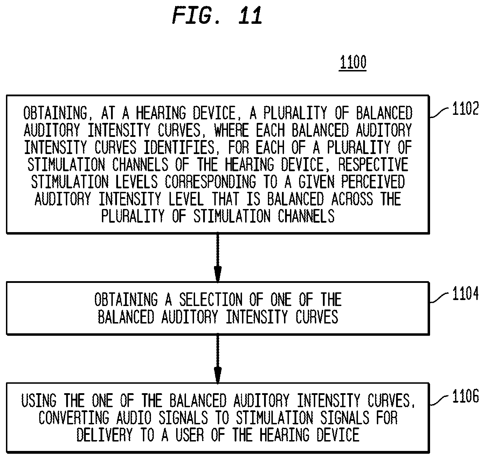

FIG. 11 is a flowchart of an example method 1100, in accordance with embodiments presented herein. Method 1100 begins at 1102 where a plurality of balanced auditory intensity curves are obtained, where each balanced auditory intensity curve identifies, for each of a plurality of stimulation channels of a hearing device, respective stimulation levels corresponding to a given perceived auditory intensity level that is balanced across the plurality of stimulation channels. At 1104, a selection of one of the balanced auditory intensity curves is obtained. At 1106, using the one of the balanced auditory intensity curves, audio signals are converted to stimulation signals for delivery to a user of the hearing device.

As should be appreciated, while particular uses of the technology have been illustrated and discussed above, the disclosed technology can be used with a variety of devices in accordance with many examples of the technology. The above discussion is not meant to suggest that the disclosed technology is only suitable for implementation within systems akin to that illustrated in the figures. In general, additional configurations can be used to practice the processes and systems herein and/or some aspects described can be excluded without departing from the processes and systems disclosed herein.

This disclosure described some aspects of the present technology with reference to the accompanying drawings, in which only some of the possible aspects were shown. Other aspects can, however, be embodied in many different forms and should not be construed as limited to the aspects set forth herein. Rather, these aspects were provided so that this disclosure was thorough and complete and fully conveyed the scope of the possible aspects to those skilled in the art.

As should be appreciated, the various aspects (e.g., portions, components, etc.) described with respect to the figures herein are not intended to limit the systems and processes to the particular aspects described. Accordingly, additional configurations can be used to practice the methods and systems herein and/or some aspects described can be excluded without departing from the methods and systems disclosed herein.

According to certain aspects, systems and non-transitory computer readable storage media are provided. The systems are configured with hardware configured to execute operations analogous to the methods of the present disclosure. The one or more non-transitory computer readable storage media comprise instructions that, when executed by one or more processors, cause the one or more processors to execute operations analogous to the methods of the present disclosure.

Similarly, where steps of a process are disclosed, those steps are described for purposes of illustrating the present methods and systems and are not intended to limit the disclosure to a particular sequence of steps. For example, the steps can be performed in differing order, two or more steps can be performed concurrently, additional steps can be performed, and disclosed steps can be excluded without departing from the present disclosure. Further, the disclosed processes can be repeated.

Although specific aspects were described herein, the scope of the technology is not limited to those specific aspects. One skilled in the art will recognize other aspects or improvements that are within the scope of the present technology. Therefore, the specific structure, acts, or media are disclosed only as illustrative aspects. The scope of the technology is defined by the following claims and any equivalents therein.

It is also to be appreciated that the embodiments presented herein are not mutually exclusive and that the various embodiments may be combined with another in any of a number of different manners.

## Claims

1. One or more non-transitory computer readable storage media, **characterized in that** the one or more non-transitory computer-readable storage media comprise instructions that, when executed by a processor (883), cause the processor (883) to:
obtain, from a plurality of first users, for each channel of a plurality of channels, one or more indications of a first plurality of perceived auditory intensity levels corresponding to a first plurality of electrical stimulus levels;
based on the one or more indications of the first plurality of perceived auditory intensity levels, generate one or more probability density functions that indicate one or more probabilities that the first plurality of electrical stimulus levels correspond to the first plurality of perceived auditory intensity levels; and
based on the one or more probability density functions, construct a plurality of perceived auditory intensity balance curves (310(1)-310(4), 675, 730) for a second user, wherein each perceived auditory intensity balance curve identifies, for each channel of the plurality of channels, respective electrical stimulus levels each corresponding to a given perceived auditory intensity level that is constant across the plurality of channels, wherein, for each perceived auditory intensity balance curve, the given perceived auditory intensity level is different from the given auditory intensity levels of other ones of the plurality of perceived auditory intensity balance curves (310(1)-310(4), 675, 730).

2. The one or more non-transitory computer readable storage media of claim 1, wherein the instructions further cause the processor (883) to:
based on the one or more indications of the first plurality of perceived auditory intensity levels, generate a plurality of probability density functions including the one or more probability density functions;
based on the plurality of probability density functions, construct a plurality of probabilistic perceived auditory intensity balance curves; and
select, for the second user, the plurality of perceived auditory intensity balance curves (310(1)-310(4), 675, 730) from among the plurality of probabilistic perceived auditory intensity balance curves.

3. The one or more non-transitory computer readable storage media of claims 1 or 2, wherein the instructions further cause the processor (883) to:
construct the plurality of perceived auditory intensity balance curves (310(1)-310(4), 675, 730) using an artificial intelligence process.

4. The one or more non-transitory computer readable storage media of claims 1 or 2, wherein the instructions further cause the processor (883) to:
using one or more of the plurality of perceived auditory intensity balance curves, translate test audio signals to test stimulation signals; and
in response to translating the test audio signals to the test stimulation signals, obtain a selection of one of the plurality of perceived auditory intensity balance curves.

5. The one or more non-transitory computer readable storage media of claims 1 or 2, wherein the instructions further cause the processor (883) to:
obtain a user indication to change from one of the plurality of perceived auditory intensity balance curves (310(1)-310(4), 675, 730) to another one of the plurality of perceived auditory intensity balance curves (675, 730); and
using the another one of the perceived auditory intensity balance curves (310(1)-310(4), 675, 730), translate audio signals to stimulation signals.

6. A system comprising:
a display screen;
a memory (884); and
at least one processor (883) operably coupled to the display screen and the memory (884), **characterized in that** the at least one processor (883) is configured to:
generate, on the display screen, one or more toggle buttons (630) to select a perceived auditory intensity balance curve from a plurality of perceived auditory intensity balance curves (615, 675), wherein each perceived auditory intensity balance curve (310(1)-310(4), 615, 675) identifies, for each channel of a plurality of channels, respective electrical stimulus levels, each corresponding to a given perceived auditory intensity level that is constant across the plurality of channels, wherein, for each perceived auditory intensity balance curve, the given perceived auditory intensity level is different from the given auditory intensity levels of other ones of the plurality of perceived auditory intensity balance curves (310(1)-310(4), 615, 675);
obtain, via the one or more toggle buttons (630), a selection of the perceived auditory intensity balance curve; and
using the perceived auditory intensity balance curve, translate audio signals to stimulation signals.

7. The system of claim 6, wherein the at least one processor is further configured to:
generate, on the display screen, a plot (605) of the plurality of perceived auditory intensity balance curves (310(1)-310(4), 615, 675).

8. The system of claims 6 or 7, wherein the at least one processor (883) is further configured to:
generate, on the display screen, a plot overlay (655) that displays a plurality of probabilistic perceived auditory intensity balance curves constructed from a plurality of probability density functions that indicate one or more probabilities that the respective electrical stimulus levels correspond to one or more given perceived auditory intensity levels.

9. The system of claim 8, wherein the at least one processor (883) is further configured to:
generate the plot with a vertical axis displayed in units of the perceived auditory intensity levels, such that the plot displays the probabilistic perceived auditory intensity balance curves as horizontal lines.

10. The system of claims 6 or 7, wherein the at least one processor (883) is further configured to:
generate the plot with a vertical axis displayed in units of the electrical stimulus levels.

11. The system of claims 6 or 7, wherein the at least one processor (883) is further configured to:
construct the plurality of perceived auditory intensity balance curves (310(1)-310(4), 615, 675) using an artificial intelligence process.

12. The system of any one of claims 6 to 11, wherein the at least one processor (883) is further configured to:
for each of a plurality of stimulation channels of a hearing device, obtain perceived auditory intensity levels of a plurality of stimulation signals delivered to a user of a hearing device via a corresponding stimulation channel; and
construct the plurality of perceived auditory intensity balance curves (615, 675).

13. The system of claim 12, wherein the at least one processor (883) is further configured to:
construct the plurality of balanced auditory intensity curves (615, 675) based on one or more probability density functions that indicate probabilities that one or more stimulation levels correspond to one or more perceived auditory intensity levels.

14. The system of claim 12 or 13, wherein the at least one processor (883) is further configured to:
administer a broadband loudness-scaling test to the user of the hearing device; and
based on the broadband loudness-scaling test, synchronize the hearing device with another hearing device.

## Patentansprüche

1. Ein oder mehrere nicht-transitorische computerlesbare Speichermedien, **dadurch gekennzeichnet, dass** das eine oder die mehreren nicht-transitorischen computerlesbaren Speichermedien Anweisungen umfassen, die, wenn sie von einem Prozessor (883) ausgeführt werden, den Prozessor (883) dazu veranlassen:
von einer Vielzahl erster Benutzer für jeden Kanal einer Vielzahl von Kanälen eine oder mehrere Angaben zu einer ersten Vielzahl wahrgenommener Hörintensitätspegel zu erhalten, die einer ersten Vielzahl elektrischer Stimulationspegel entsprechen;
auf Grundlage der einen oder mehreren Angaben zu der ersten Vielzahl wahrgenommener Hörintensitätspegel eine oder mehrere Wahrscheinlichkeitsdichtefunktionen zu erzeugen, die eine oder mehrere Wahrscheinlichkeiten angeben, dass die erste Vielzahl elektrischer Stimulationspegel der ersten Vielzahl wahrgenommener Hörintensitätspegel entspricht; und
auf Grundlage der einen oder mehreren Wahrscheinlichkeitsdichtefunktionen eine Vielzahl von Ausgleichskurven der wahrgenommenen Hörintensität (310(1)-310(4), 675, 730) für einen zweiten Benutzer zu erstellen, wobei jede Ausgleichskurve der wahrgenommenen Hörintensität für jeden Kanal der Vielzahl von Kanälen jeweilige elektrische Stimulationspegel identifiziert, von denen jeder einem bestimmten wahrgenommenen Hörintensitätspegel entspricht, der über die Vielzahl von Kanälen konstant ist, wobei für jede Ausgleichskurve der wahrgenommenen Hörintensität der bestimmte wahrgenommene Hörintensitätspegel von den bestimmten Hörintensitätspegeln anderer der Vielzahl von Ausgleichskurven der wahrgenommenen Hörintensität (310(1)-310(4), 675, 730) verschieden ist.

2. Ein oder mehrere nicht-transitorische computerlesbare Speichermedien nach Anspruch 1, wobei die Anweisungen den Prozessor (883) ferner dazu veranlassen:
auf Grundlage der einen oder mehreren Angaben zu der ersten Vielzahl wahrgenommener Hörintensitätspegel eine Vielzahl von Wahrscheinlichkeitsdichtefunktionen zu erzeugen, welche die eine oder die mehreren Wahrscheinlichkeitsdichtefunktionen einschließt;
auf Grundlage der Vielzahl von Wahrscheinlichkeitsdichtefunktionen eine Vielzahl probabilistischer Ausgleichskurven der wahrgenommenen Hörintensität zu erstellen; und
für den zweiten Benutzer die Vielzahl von Ausgleichskurven der wahrgenommenen Hörintensität (310(1)-310(4), 675, 730) aus der Vielzahl probabilistischer Ausgleichskurven der wahrgenommenen Hörintensität auszuwählen.

3. Ein oder mehrere nicht-transitorische computerlesbare Speichermedien nach Anspruch 1 oder 2, wobei die Anweisungen den Prozessor (883) ferner dazu veranlassen:
die Vielzahl von Ausgleichskurven der wahrgenommenen Hörintensität (310(1)-310(4), 675, 730) unter Verwendung eines Verfahrens der künstlichen Intelligenz zu erstellen.

4. Ein oder mehrere nicht-transitorische computerlesbare Speichermedien nach Anspruch 1 oder 2, wobei die Anweisungen den Prozessor (883) ferner dazu veranlassen:
unter Verwendung einer oder mehrerer der Vielzahl von Ausgleichskurven der wahrgenommenen Hörintensität Testaudiosignale in Teststimulationssignale umzusetzen; und
als Reaktion auf das Umsetzen der Testaudiosignale in die Teststimulationssignale eine Auswahl einer der Vielzahl von Ausgleichskurven der wahrgenommenen Hörintensität zu erhalten.

5. Ein oder mehrere nicht-transitorische computerlesbare Speichermedien nach Anspruch 1 oder 2, wobei die Anweisungen den Prozessor (883) ferner dazu veranlassen:
eine Benutzerangabe zum Wechsel von einer der Vielzahl von Ausgleichskurven der wahrgenommenen Hörintensität (310(1)-310(4), 675, 730) zu einer anderen der Vielzahl von Ausgleichskurven der wahrgenommenen Hörintensität (675, 730) zu erhalten; und
unter Verwendung der anderen einen der Ausgleichskurven der wahrgenommenen Hörintensität (310(1)-310(4), 675, 730) Audiosignale in Stimulationssignale umzusetzen.

6. System, umfassend:
einen Anzeigebildschirm;
einen Speicher (884); und
mindestens einen Prozessor (883), der funktional mit dem Anzeigebildschirm und dem Speicher (884) gekoppelt ist, **dadurch gekennzeichnet, dass** der mindestens eine Prozessor (883) dazu konfiguriert ist:
auf dem Anzeigebildschirm eine oder mehrere Umschaltflächen (630) zu erzeugen, um eine Ausgleichskurve der wahrgenommenen Hörintensität aus einer Vielzahl von Ausgleichskurven der wahrgenommenen Hörintensität (615, 675) auszuwählen, wobei jede Ausgleichskurve der wahrgenommenen Hörintensität (310(1)-310(4), 615, 675) für jeden Kanal einer Vielzahl von Kanälen jeweilige elektrische Stimulationspegel identifiziert, von denen jeder einem bestimmten wahrgenommenen Hörintensitätspegel entspricht, der über die Vielzahl von Kanälen konstant ist, wobei für jede Ausgleichskurve der wahrgenommenen Hörintensität der bestimmte wahrgenommene Hörintensitätspegel von den bestimmten Hörintensitätspegeln anderer der Vielzahl von Ausgleichskurven der wahrgenommenen Hörintensität (310(1)-310(4), 615, 675) verschieden ist;
über die eine oder die mehreren Umschaltflächen (630) eine Auswahl der Ausgleichskurve der wahrgenommenen Hörintensität zu erhalten; und
unter Verwendung der Ausgleichskurve der wahrgenommenen Hörintensität Audiosignale in Stimulationssignale umzusetzen.

7. System nach Anspruch 6, wobei der mindestens eine Prozessor ferner dazu konfiguriert ist:
auf dem Anzeigebildschirm ein Diagramm (605) der Vielzahl von Ausgleichskurven der wahrgenommenen Hörintensität (310(1)-310(4), 615, 675) zu erzeugen.

8. System nach Anspruch 6 oder 7, wobei der mindestens eine Prozessor (883) ferner dazu konfiguriert ist:
auf dem Anzeigebildschirm eine Diagrammüberlagerung (655) zu erzeugen, die eine Vielzahl probabilistischer Ausgleichskurven der wahrgenommenen Hörintensität darstellt, die aus einer Vielzahl von Wahrscheinlichkeitsdichtefunktionen erstellt sind, welche eine oder mehrere Wahrscheinlichkeiten angeben, dass die jeweiligen elektrischen Stimulationspegel einem oder mehreren bestimmten wahrgenommenen Hörintensitätspegeln entsprechen.

9. System nach Anspruch 8, wobei der mindestens eine Prozessor (883) ferner dazu konfiguriert ist:
das Diagramm mit einer vertikalen Achse zu erzeugen, die in Einheiten der wahrgenommenen Hörintensitätspegel dargestellt ist, so dass das Diagramm die probabilistischen Ausgleichskurven der wahrgenommenen Hörintensität als horizontale Linien darstellt.

10. System nach Anspruch 6 oder 7, wobei der mindestens eine Prozessor (883) ferner dazu konfiguriert ist:
das Diagramm mit einer vertikalen Achse zu erzeugen, die in Einheiten der elektrischen Stimulationspegel dargestellt ist.

11. System nach Anspruch 6 oder 7, wobei der mindestens eine Prozessor (883) ferner dazu konfiguriert ist:
die Vielzahl von Ausgleichskurven der wahrgenommenen Hörintensität (310(1)-310(4), 615, 675) unter Verwendung eines Verfahrens der künstlichen Intelligenz zu erstellen.

12. System nach einem der Ansprüche 6 bis 11, wobei der mindestens eine Prozessor (883) ferner dazu konfiguriert ist:
für jeden einer Vielzahl von Stimulationskanälen eines Hörgeräts wahrgenommene Hörintensitätspegel einer Vielzahl von Stimulationssignalen zu erhalten, die einem Benutzer eines Hörgeräts über einen entsprechenden Stimulationskanal zugeführt werden; und
die Vielzahl von Ausgleichskurven der wahrgenommenen Hörintensität (615, 675) zu erstellen.

13. System nach Anspruch 12, wobei der mindestens eine Prozessor (883) ferner dazu konfiguriert ist:
die Vielzahl ausgeglichener Hörintensitätskurven (615, 675) auf Grundlage einer oder mehrerer Wahrscheinlichkeitsdichtefunktionen zu erstellen, die Wahrscheinlichkeiten angeben, dass ein oder mehrere Stimulationspegel einem oder mehreren wahrgenommenen Hörintensitätspegeln entsprechen.

14. System nach Anspruch 12 oder 13, wobei der mindestens eine Prozessor (883) ferner dazu konfiguriert ist:
bei dem Benutzer des Hörgeräts einen Breitband-Lautheitsskalierungstest durchzuführen; und
auf Grundlage des Breitband-Lautheitsskalierungstests das Hörgerät mit einem anderen Hörgerät zu synchronisieren.

## Revendications

1. Un ou plusieurs supports de stockage non transitoires lisibles par ordinateur, **caractérisés en ce que** le ou les supports de stockage non transitoires lisibles par ordinateur comprennent des instructions qui, lorsqu'elles sont exécutées par un processeur (883), amènent le processeur (883) à :
obtenir, auprès d'une pluralité de premiers utilisateurs, pour chaque canal d'une pluralité de canaux, une ou plusieurs indications d'une première pluralité de niveaux d'intensité auditive perçue correspondant à une première pluralité de niveaux de stimulation électrique ;
sur la base de la ou des indications de la première pluralité de niveaux d'intensité auditive perçue, générer une ou plusieurs fonctions de densité de probabilité qui indiquent une ou plusieurs probabilités selon lesquelles la première pluralité de niveaux de stimulation électrique correspond à la première pluralité de niveaux d'intensité auditive perçue ; et
sur la base de la ou des fonctions de densité de probabilité, construire une pluralité de courbes d'équilibrage de l'intensité auditive perçue (310(1)-310(4), 675, 730) pour un second utilisateur, chaque courbe d'équilibrage de l'intensité auditive perçue identifiant, pour chaque canal de la pluralité de canaux, des niveaux respectifs de stimulation électrique correspondant chacun à un niveau donné d'intensité auditive perçue qui est constant sur l'ensemble de la pluralité de canaux, dans lequel, pour chaque courbe d'équilibrage de l'intensité auditive perçue, le niveau donné d'intensité auditive perçue est différent des niveaux donnés d'intensité auditive d'autres courbes de la pluralité de courbes d'équilibrage de l'intensité auditive perçue (310(1)-310(4), 675, 730).

2. Un ou plusieurs supports de stockage non transitoires lisibles par ordinateur selon la revendication 1, dans lesquels les instructions amènent en outre le processeur (883) à :
sur la base de la ou des indications de la première pluralité de niveaux d'intensité auditive perçue, générer une pluralité de fonctions de densité de probabilité comprenant la ou les fonctions de densité de probabilité ;
sur la base de la pluralité de fonctions de densité de probabilité, construire une pluralité de courbes probabilistes d'équilibrage de l'intensité auditive perçue ; et
sélectionner, pour le second utilisateur, la pluralité de courbes d'équilibrage de l'intensité auditive perçue (310(1)-310(4), 675, 730) parmi la pluralité de courbes probabilistes d'équilibrage de l'intensité auditive perçue.

3. Un ou plusieurs supports de stockage non transitoires lisibles par ordinateur selon la revendication 1 ou 2, dans lesquels les instructions amènent en outre le processeur (883) à :
construire la pluralité de courbes d'équilibrage de l'intensité auditive perçue (310(1)-310(4), 675, 730) en utilisant un processus d'intelligence artificielle.

4. Un ou plusieurs supports de stockage non transitoires lisibles par ordinateur selon la revendication 1 ou 2, dans lesquels les instructions amènent en outre le processeur (883) à :
en utilisant une ou plusieurs de la pluralité de courbes d'équilibrage de l'intensité auditive perçue, convertir des signaux audio de test en signaux de stimulation de test ; et
en réponse à la conversion des signaux audio de test en signaux de stimulation de test, obtenir une sélection de l'une de la pluralité de courbes d'équilibrage de l'intensité auditive perçue.

5. Un ou plusieurs supports de stockage non transitoires lisibles par ordinateur selon la revendication 1 ou 2, dans lesquels les instructions amènent en outre le processeur (883) à :
obtenir une indication de l'utilisateur pour passer de l'une de la pluralité de courbes d'équilibrage de l'intensité auditive perçue (310(1)-310(4), 675, 730) à une autre de la pluralité de courbes d'équilibrage de l'intensité auditive perçue (675, 730) ; et
en utilisant l'autre une des courbes d'équilibrage de l'intensité auditive perçue (310(1)-310(4), 675, 730), convertir des signaux audio en signaux de stimulation.

6. Système comprenant :
un écran d'affichage ;
une mémoire (884) ; et
au moins un processeur (883) couplé de manière opérationnelle à l'écran d'affichage et à la mémoire (884), **caractérisé en ce que** ledit au moins un processeur (883) est configuré pour :
générer, sur l'écran d'affichage, un ou plusieurs boutons de basculement (630) pour sélectionner une courbe d'équilibrage de l'intensité auditive perçue parmi une pluralité de courbes d'équilibrage de l'intensité auditive perçue (615, 675), chaque courbe d'équilibrage de l'intensité auditive perçue (310(1)-310(4), 615, 675) identifiant, pour chaque canal d'une pluralité de canaux, des niveaux respectifs de stimulation électrique correspondant chacun à un niveau donné d'intensité auditive perçue qui est constant sur l'ensemble de la pluralité de canaux, dans lequel, pour chaque courbe d'équilibrage de l'intensité auditive perçue, le niveau donné d'intensité auditive perçue est différent des niveaux donnés d'intensité auditive d'autres courbes de la pluralité de courbes d'équilibrage de l'intensité auditive perçue (310(1)-310(4), 615, 675) ;
obtenir, par l'intermédiaire du ou des boutons de basculement (630), une sélection de la courbe d'équilibrage de l'intensité auditive perçue ; et
en utilisant la courbe d'équilibrage de l'intensité auditive perçue, convertir des signaux audio en signaux de stimulation.

7. Système selon la revendication 6, dans lequel l'au moins un processeur est en outre configuré pour :
générer, sur l'écran d'affichage, un graphique (605) de la pluralité de courbes d'équilibrage de l'intensité auditive perçue (310(1)-310(4), 615, 675).

8. Système selon la revendication 6 ou 7, dans lequel l'au moins un processeur (883) est en outre configuré pour :
générer, sur l'écran d'affichage, une superposition de graphique (655) qui affiche une pluralité de courbes probabilistes d'équilibrage de l'intensité auditive perçue construites à partir d'une pluralité de fonctions de densité de probabilité qui indiquent une ou plusieurs probabilités selon lesquelles les niveaux respectifs de stimulation électrique correspondent à un ou plusieurs niveaux donnés d'intensité auditive perçue.

9. Système selon la revendication 8, dans lequel l'au moins un processeur (883) est en outre configuré pour :
générer le graphique avec un axe vertical affiché en unités des niveaux d'intensité auditive perçue, de telle sorte que le graphique affiche les courbes probabilistes d'équilibrage de l'intensité auditive perçue sous forme de lignes horizontales.

10. Système selon la revendication 6 ou 7, dans lequel l'au moins un processeur (883) est en outre configuré pour :
générer le graphique avec un axe vertical affiché en unités des niveaux de stimulation électrique.

11. Système selon la revendication 6 ou 7, dans lequel l'au moins un processeur (883) est en outre configuré pour :
construire la pluralité de courbes d'équilibrage de l'intensité auditive perçue (310(1)-310(4), 615, 675) en utilisant un processus d'intelligence artificielle.

12. Système selon l'une quelconque des revendications 6 à 11, dans lequel l'au moins un processeur (883) est en outre configuré pour :
pour chacun d'une pluralité de canaux de stimulation d'un dispositif auditif, obtenir des niveaux d'intensité auditive perçue d'une pluralité de signaux de stimulation délivrés à un utilisateur d'un dispositif auditif par l'intermédiaire d'un canal de stimulation correspondant ; et
construire la pluralité de courbes d'équilibrage de l'intensité auditive perçue (615, 675).

13. Système selon la revendication 12, dans lequel l'au moins un processeur (883) est en outre configuré pour :
construire la pluralité de courbes d'intensité auditive équilibrées (615, 675) sur la base d'une ou plusieurs fonctions de densité de probabilité qui indiquent des probabilités selon lesquelles un ou plusieurs niveaux de stimulation correspondent à un ou plusieurs niveaux d'intensité auditive perçue.

14. Système selon la revendication 12 ou 13, dans lequel l'au moins un processeur (883) est en outre configuré pour :
administrer un test de mise à l'échelle de la sonie à large bande à l'utilisateur du dispositif auditif ; et
sur la base du test de mise à l'échelle de la sonie à large bande, synchroniser le dispositif auditif avec un autre dispositif auditif.
